# EUROPEAN PATENT APPLICATION

(11) **EP 2 221 372 A1**
(43) Date of publication of application: **25.08.2010**
(21) Application number: 08852930.0
(22) Date of filing: 24.09.2008
(51) Int. Cl.: C12N 15/09

(54) **METHOD OF SITE-SELECTIVELY CLEAVING TARGET NUCLEIC ACID**

(30) Priority: 19.11.2007 JP 2007299747
(71) Applicant: The University of Tokyo, Bunkyo-Ku Tokyo 113-8654 (JP)
(72) Inventor: KOMIYAMA, Makoto, Tokyo 113-8654 (JP); LONNBERG, Tuomas, Tokyo 113-8654 (JP); SUZUKI, Yuta, Tokyo 113-8654 (JP)
(74) Representative: Denison, Christopher Marcus
(86) International application number: PCT/JP2008/067785
(87) International publication number: WO 2009/066513

(57) **Abstract**

There is provided a method for cleaving a target nucleic acid at a desired site using a metal ion or a metal ion complex as a catalyst for cleaving a nucleic acid (DNA, etc.), which has high site-selectivity, high reaction efficiency and low side-reactivity (non-specific reactivity), and is economical and convenient. The method for cleaving a target nucleic acid of the present invention comprises allowing a target nucleic acid to come into contact with a specific complex compound and a metal ion or a metal complex, or allowing a target nucleic acid to come into contact with a specific complex compound, to which a metal ion or a metal complex binds.

SEQUENCE LISTING

<110> THE UNIVERSITY OF TOKYO

<120> A method of cleaving a nucleic acid site-selectively

<130> PCT08-0059

<150> JP 2007-299747
<151> 2007-11-19

<160> 4

<170> PatentIn version 3.4

<210> 1
<211> 20
<212> DNA
<213> Artificial

<220>
<223> synthetic DNA

<400> 1
gccattcctg attctaattg 20


<210> 2
<211> 20
<212> DNA
<213> Artificial

<220>
<223> synthetic DNA

<400> 2
cacgtctgac agctggattc 20


<210> 3
<211> 85
<212> DNA
<213> Artificial

<220>
<223> synthetic DNA

<400> 3
gaactggacc tctagctcct caattagaat caggaatggc ttatggtgca gactgtcgac 60

ctaagtagac gcaatgtcgg acgta                                                85

<210>  4
<211>  41
<212>  DNA
<213>  Artificial

<220>
<223>  synthetic DNA

<220>
<221>  misc_feature
<222>  (21)..(21)
<223>  n is a, c, g, or t

<400>  4
caattagaat caggaatggc ngtgcagact gtcgacctaa g                              41

## Description

### TECHNICAL FIELD

The present invention relates to: a method for site-selectively cleaving a nucleic acid; and a compound, a reagent, and a kit used for the method.

### BACKGROUND ART

A method for cleaving a DNA strand at any given site and then binding foreign DNA to the cleaved site is anticipated to play an important role in various fields for dealing with genes, such as medical and molecular biological fields. In general, restriction enzyme is widely used to cleave a DNA strand. However, restriction enzyme has low specificity for nucleotide sequences, and thus it is not able to selectively cleave enormous DNA at a specific site. In addition, as a method for cleaving DNA that does not use restriction enzymes, an attempt has been made to cleave DNA with a metal ion or a metal ion complex. For example, a cerium(IV) ion or the like has been known to have DNA cleavage ability. However, this cleavage method has almost no specificity for nucleotide sequences, and thus it has been difficult for this method to cleave enormous DNA at a specific site.

In contrast, in recent years, a method capable of cleaving enormous DNA at a specific site using a metal ion or a metal ion complex has been developed (see JP Patent Publication (Kokai) Nos. 2005-143484 A and 2006-174702 A, for example).

### DISCLOSURE OF THE INVENTION

According to the conventional method, it has become possible to cleave enormous DNA at a desired site using a metal ion or the like. However, in order to achieve higher site-selectivity and lower side reactivity (non-specific reactivity), it has been desired to develop a more excellent method. Moreover, in terms of economic efficiency and convenience as well, a highly practical method has been desired.

Hence, it is an object of the present invention to provide a method for cleaving a target nucleic acid at a desired site using a metal ion or a metal ion complex as a catalyst for cleaving a nucleic acid (DNA, etc.), which has high site-selectivity, high reaction efficiency and low side-reactivity (non-specific reactivity), and is economical and convenient. It is another object of the present invention to provide a novel complex compound, a reagent, and a kit, which are used for the aforementioned cleavage method.

The present inventor has conducted intensive studies directed towards achieving the aforementioned objects. As a result, the inventor has completed the present invention.

Specifically, the present invention has the following features.
(1) A complex compound, wherein a compound containing multiple phosphono groups binds to a compound binding to a specific nucleic acid sequence via a linker portion containing an O-alkyloxime group.
   The complex compound of the present invention is, for example, a complex compound, wherein the above-described nucleic acid (nucleic acid sequence) is DNA (DNA sequence).
   In the complex compound of the present invention, the above-described compound binding to a specific nucleic acid sequence is an oligonucleotide or a peptide nucleic acid, for example. Herein, the number of bases of the oligonucleotide is 7 to 50, for example, or the number of bases of the peptide nucleic acid is 5 to 25, for example. The complex compound of the present invention includes, for example, a complex compound represented by the following formula (1): 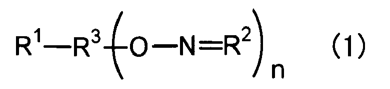 (wherein R¹ represents a compound binding to a specific nucleic acid sequence, R² represents a compound containing multiple phosphono groups, R³ represents a single bond or any given group, and n represents an integer of 1 to 10).
   Herein, in the above-described formula (1), for example, R¹ may be an oligonucleotide or a peptide nucleic acid, and R³ may be a group represented by the following formula (3): 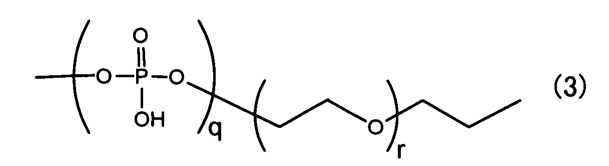 (wherein q represents an integer of 0 to 2, and r represents an integer of 0 to 30). Moreover, in the above-described formula (1), R² may be represented by the following formula (2): 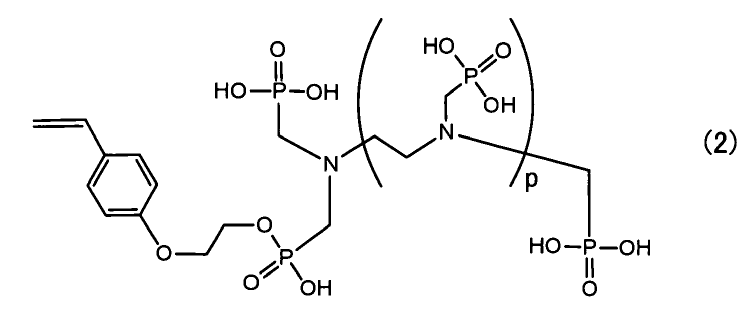 (wherein p represents an integer of 0 to 3).
   The complex compound of the present invention is, for example, a complex compound, wherein a metal ion or a metal complex binds to the above-described phosphono group. Herein, the metal ion or the metal complex is, for example, a cerium(IV) ion or a cerium(IV) complex, or a cerium(III) ion or a cerium(III) complex, respectively. In particular, such complex is, for example, a complex of cerium(IV) or cerium(III) and polyamine-N-polycarboxylic acid.
(2) A method for cleaving a target nucleic acid, which comprises allowing a target nucleic acid to come into contact with the complex compound according to (1) above (except for a compound to which a metal ion or the like binds) and a metal ion or a metal complex. Herein, the metal ion or the metal complex is, for example, a cerium(IV) ion or a cerium(IV) complex, or a cerium(III) ion or a cerium(III) complex, respectively. In particular, such complex is, for example, a complex of cerium(IV) or cerium(III) and polyamine-N-polycarboxylic acid.
   The method according to (2) above is, for example, a method, which comprises oxidizing the cerium(III) ion or the cerium(III) complex, before and/or after it is allowed to come into contact with the target nucleic acid, so as to convert it to a cerium(IV) ion or a cerium(IV) complex.
(3) A method for cleaving a target nucleic acid, which comprises allowing a target nucleic acid to come into contact with the complex compound according to (1) above (a compound to which a metal ion or the like binds).
   The method according to (3) above is, for example, a method, wherein the metal ion or the metal complex in the complex compound is a cerium(III) ion or a cerium(III) complex, and wherein the cerium(III) ion or the cerium(III) complex is oxidized before and/or after it is allowed to come into contact with the target nucleic acid, so as to convert it to a cerium(IV) ion or a cerium(IV) complex.
   In the method for cleaving a target nucleic acid of the present invention ((2) and (3) above; the same applies below), the target nucleic acid is, for example, DNA.
   The method for cleaving a target nucleic acid of the present invention is, for example, a method, wherein a complex compound (a) binding to a 5'-terminal region in the target portion of the target nucleic acid and a complex compound (b) binding to a 3'-terminal region in the target portion thereof are used as the above-described complex compounds. Herein, it is preferable that a gap be present between the 5'-terminal region of the target nucleic acid, to which the complex compound (a) binds, and the 3'-terminal region of the target nucleic acid, to which the complex compound (b) binds, and that a desired cleavage point be present in the gap. Moreover, portions in the complex compound (a) and the complex compound (b), which bind to the target portions, are oligonucleotides or peptide nucleic acids, for example.
   The method for cleaving a target nucleic acid of the present invention is, for example, a method, wherein the above-described target nucleic acid is double-stranded, and a complex compound (A) binding to the target portion on one strand of the target nucleic acid and a complex compound (B) binding to the target portion on the other strand of the target nucleic acid are used as the above-described complex compounds. Herein, portions in the complex compound (A) and the complex compound (B), which bind to the target portions, are oligonucleotides or peptide nucleic acids, for example. Moreover, portions in the complex compound (A) and the complex compound (B), which bind to the target portions, have portions complementary to each other and also have portions that are not complementary to each other on the 5'-terminal and/or 3'-terminal sides thereof, for example.
(4) A reagent for cleaving a target nucleic acid, which comprises the complex compound according to (1) above (excluding a compound to which a metal ion or the like binds) and a metal ion or a metal complex. Herein, the metal ion or the metal complex is, for example, a cerium(IV) ion or a cerium(IV) complex, or a cerium(III) ion or a cerium(III) complex, respectively. In particular, such complex is, for example, a complex of cerium(IV) or cerium(III) and polyamine-N-polycarboxylic acid.
(5) A reagent for cleaving a target nucleic acid, which comprises the complex compound according to (1) above (a compound to which a metal ion or the like binds).

In the reagent of the present invention ((4) and (5) above; the same applies below), the target nucleic acid is, for example, DNA.

The reagent of the present invention is, for example, a reagent, which comprises a complex compound (a) binding to a 5'-terminal region in the target portion of the target nucleic acid and a complex compound (b) binding to a 3'-terminal region in the target portion thereof as the above-described complex compounds. Herein, it is preferable that a gap be present between the 5'-terminal region of the target nucleic acid, to which the complex compound (a) binds, and the 3'-terminal region of the target nucleic acid, to which the complex compound (b) binds, and that a desired cleavage point be present in the gap. Moreover, portions in the complex compound (a) and the complex compound (b), which bind to the target portions, are oligonucleotides or peptide nucleic acids, for example.

The reagent of the present invention is, for example, a reagent, wherein the above-described target nucleic acid is double-stranded, and a complex compound (A) binding to the target portion on one strand of the target nucleic acid and a complex compound (B) binding to the target portion on the other strand of the target nucleic acid are used as the above-described complex compounds. Herein, portions in the complex compound (A) and the complex compound (B), which bind to the target portions, are oligonucleotides or peptide nucleic acids, for example. Moreover, portions in the complex compound (A) and the complex compound (B), which bind to the target portions, have portions complementary to each other and also have portions that are not complementary to each other on the 5'-terminal and/or 3'-terminal sides thereof, for example. (6) A kit for cleaving a target nucleic acid, which comprises the reagent according to (4) or (5) above.

### BRIEF DESCRIPTION OF THE DRAWINGS

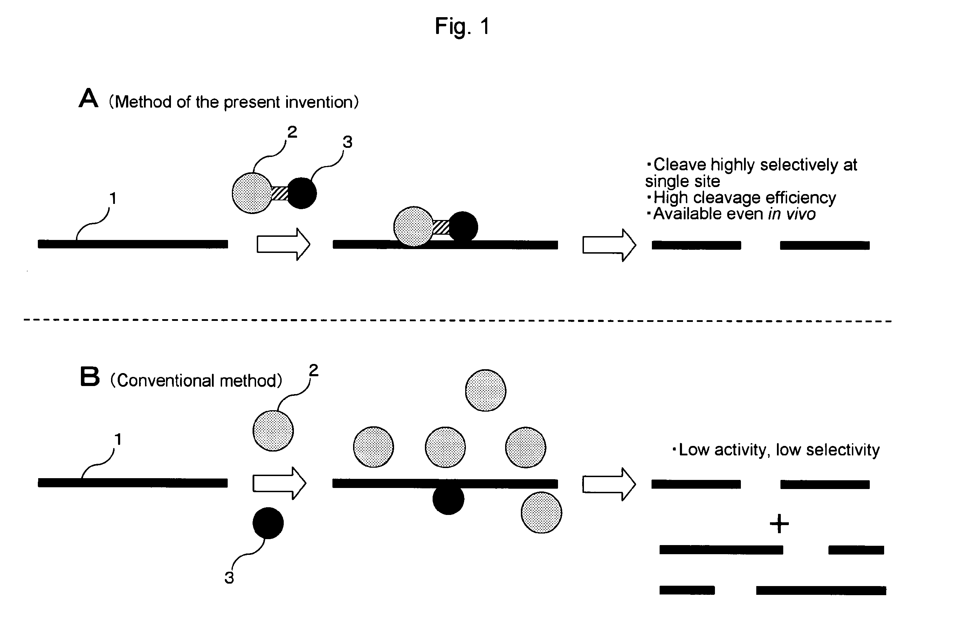

Figure 1 is a view showing a summary of the complex compound of the present invention and the cleavage of target DNA using the same. Figure 1A is a schematic view showing one embodiment of the method of the present invention, and Figure 1B is a schematic view showing one embodiment of the conventional method. In the method shown in Figure 1A, a molecule 2 binding to a desired region of target DNA is allowed to bind to a DNA-cleaving catalyst 3, and it is then used. It is to be noted that the DNA-cleaving catalyst 3 used in the method shown in Figure 1B contains 0 or 1 phosphono group (in contrast, the DNA-cleaving catalyst 3 used in the method shown in Figure 1A contains multiple (two or more) phosphono groups).

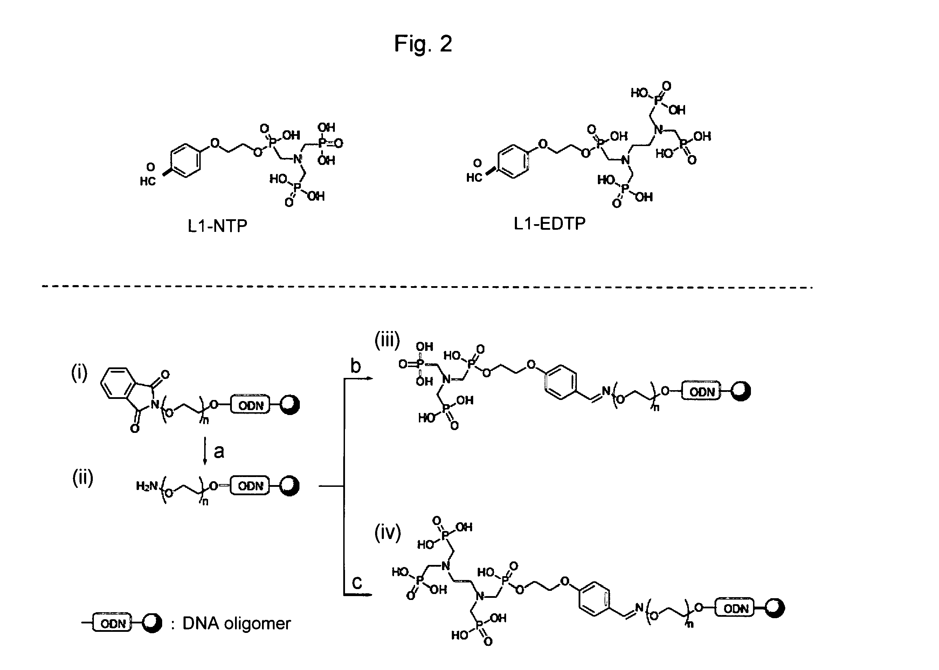

Figure 2 is a schematic view (summary) showing a synthetic example of the complex compound of the present invention.

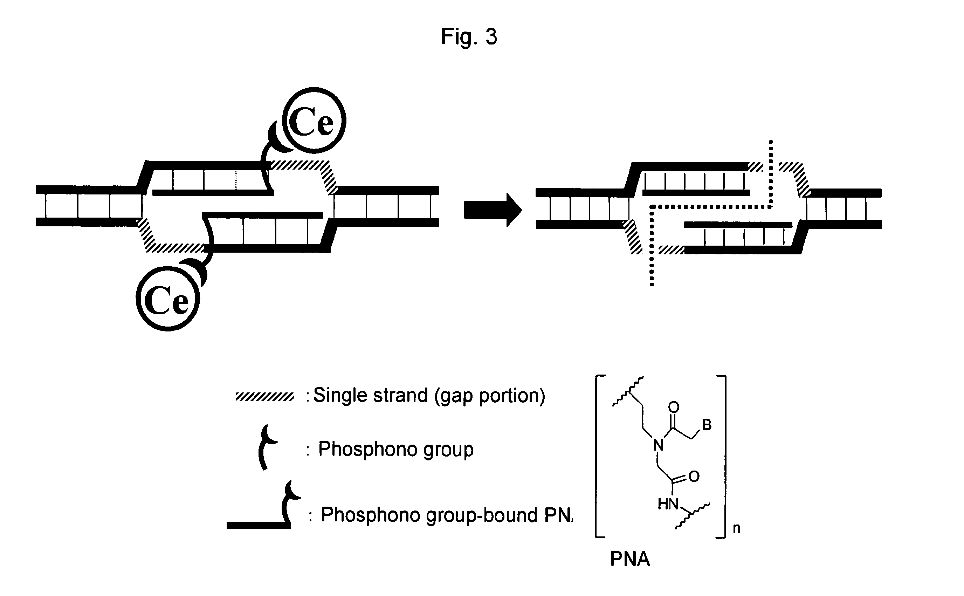

Figure 3 is a view showing a summary of the "cleavage of double-stranded DNA" using the complex compound of the present invention. Specifically, the view shows that a catalyst (Ce(IV) is concentrated at a cleavage site by binding a group containing multiple phosphono groups to a peptide nucleic acid (PNA) capable of complementarily binding to each strand of double-stranded DNA.

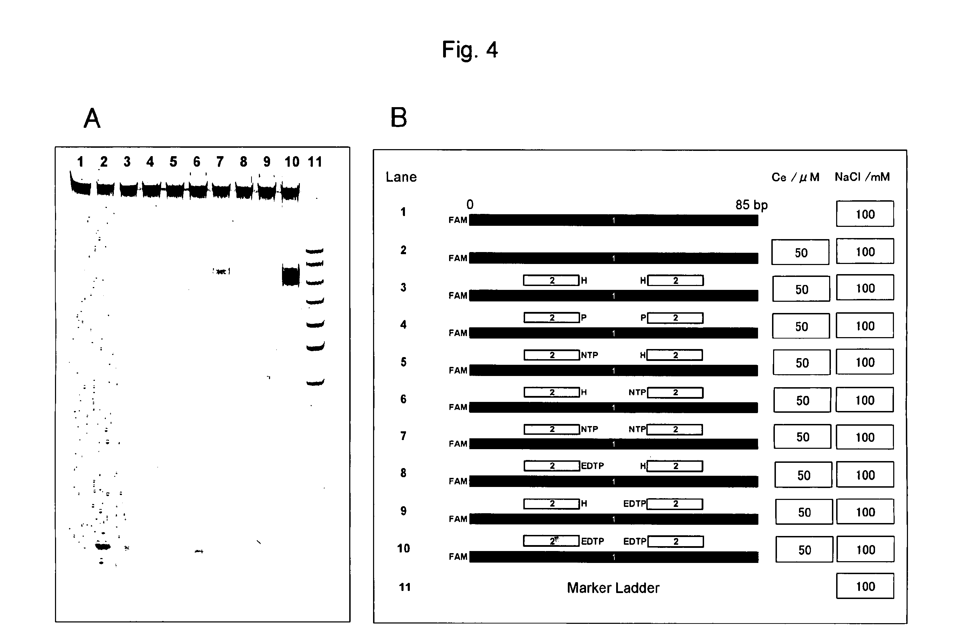

Figure 4 is a view showing the results of the site-selective cleavage (1) of target DNA using the method of the present invention (Example 2; 85 mer target with 5-base gap, t = 114 h, 50 µM incubated Ce(IV)/EDTA). The left figure (A) shows the results of 20% denatured polyacrylamide gel electrophoresis, and the right figure (B) is a view schematically showing the types of the complex compounds of the present invention used for target DNA and the combinations thereof (the types and embodiments of DNA derivatives hybridized to the target DNA). The embodiment of lane 4 exhibited the highest cleavage activity in the conventional methods.

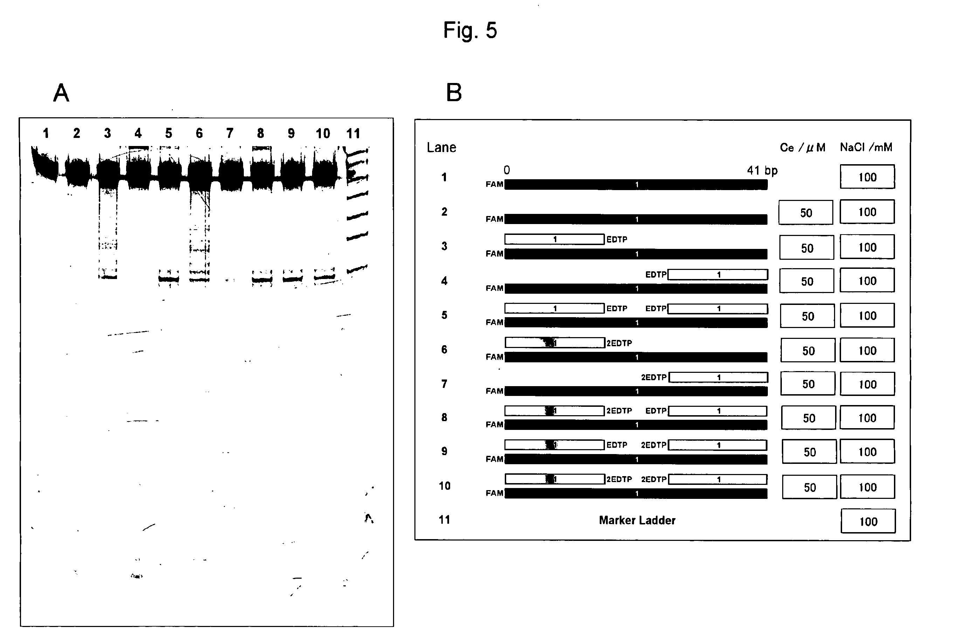

Figure 5 is a view showing the results of the site-selective cleavage (2) of target DNA using the method of the present invention (Example 3; 41 mer target with 1-base gap, t = 67 h, 20 µM non-incubated Ce(IV)/EDTA). The left figure (A) shows the results of 20% denatured polyacrylamide gel electrophoresis, and the right figure (B) is a view schematically showing the types of the complex compounds of the present invention used for target DNA and the combinations thereof (the types and embodiments of DNA derivatives hybridized to the target DNA).

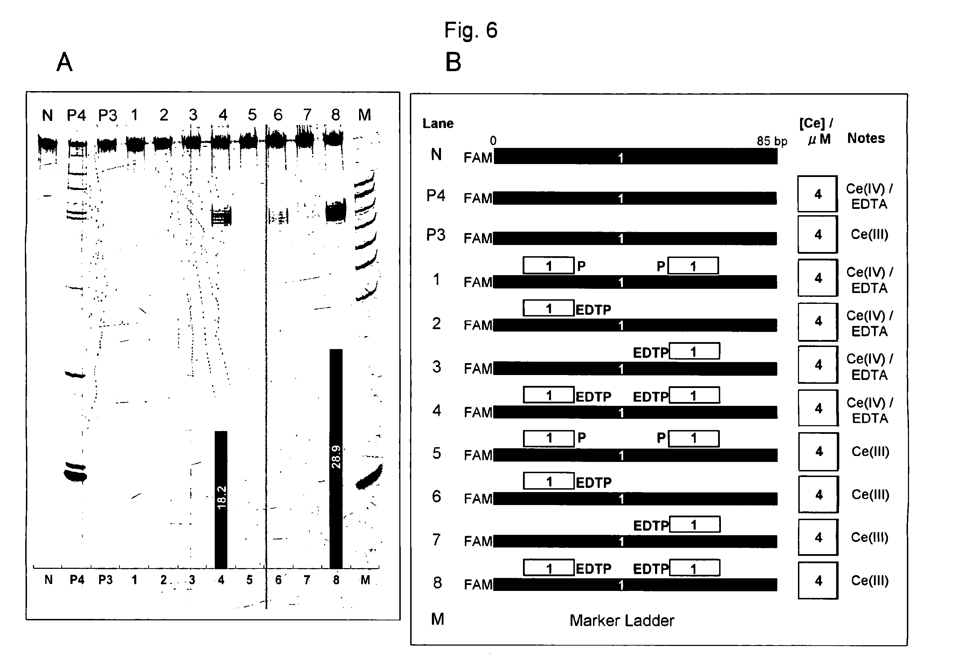

Figure 6 is a view showing the results of the site-selective cleavage (3) of target DNA using the method of the present invention (Example 4; 85 mer target with 5-base gap, t = 54 h, 4 µM incubated Ce(IV)/EDTA or Ce(III)). The left figure (A) shows the results of 20% denatured polyacrylamide gel electrophoresis, and the right figure (B) is a view schematically showing the types of the complex compounds of the present invention used for target DNA and the combinations thereof (the types and embodiments of DNA derivatives hybridized to the target DNA).

### Description of Symbols

1: Target DNA, 2: Molecule that binds to a desired region of target DNA, 3: DNA-cleaving catalyst.

### BEST MODE FOR CARRYING OUT THE INVENTION

The present invention will be described in detail below. The following descriptions are not intended to limit the scope of the present invention. Other than the following examples, the present invention may be modified and may be carried out, as appropriate, within a range that does not impair the intention of the present invention.

The present specification includes all of the contents as disclosed in the specification of Japanese Patent Application No. 2007-299747, which is a priority document of the present application. Moreover, all publications cited in the present specification, which include prior art documents and patent documents such as laid-open application publications and patent publications, are incorporated herein by reference in their entirety.

### 1. Summary of the present invention

A common method for cleaving a nucleic acid such as DNA at a predetermined site uses (1) a catalyst molecule that cleaves the nucleic acid and (2) a molecule that binds to a predetermined region (site) of the nucleic acid and activates it. As previously clarified by the present inventor, a cerium(IV) ion and a complex thereof are extremely useful as the molecules (1). However, to date, there had not been an appropriate method for binding such ion and the complex thereof to the predetermined region of the nucleic acid. Thus, the above-described molecules (1) and (2) had been independently added to a reaction system, and as a result, the cleavage efficiency had been low and the site-selectivity for cleavage had also been insufficient. Moreover, a large amount of catalyst had been required for the cleavage of a nucleic acid. Furthermore, in order to selectively cleave a nucleic acid in a cell, it is necessary that both a cerium(IV) complex and a nucleic acid-recognizing molecule be introduced into the cell, separately. This technique could not be easily realized. It is an object of the present invention to solve the aforementioned problems. The present invention provides a method for efficiently cleaving a target nucleic acid at a desired site, using a complex compound that can be easily prepared in an aqueous solution and is stable therein.

In one embodiment, the present invention relates to a method for cleaving a nucleic acid, which comprises binding a cerium(IV) complex to a nucleic acid-recognizing molecule, using a linker that can be prepared in an aqueous solution and is stable therein, and then cleaving the nucleic acid with the obtained product. Thus, by fixing a cerium(IV) complex having cleavage ability on a target region in a nucleic acid, the nucleic acid can be selectively cleaved only at a desired site (see Figure 1). Moreover, another embodiment of the present invention is a method using cerium(III) (a cerium(III) ion or a cerium(III) complex) instead of cerium(IV). In a reaction system, cerium(III) reacts with oxygen or the like in the air, so that it is spontaneously oxidized to cerium(IV), thereby obtaining DNA cleavage activity.

### 2. Method for cleaving target nucleic acid

The method for cleaving a target nucleic acid of the present invention comprises allowing a target nucleic acid to come into contact with a predetermined complex compound and a metal ion or a metal complex, or allowing a target nucleic acid to come into contact with a predetermined complex compound, to which a metal ion or a metal complex binds. The type of a target nucleic acid used herein is not limited. A preferred example of such target nucleic acid is DNA (single-stranded and double-stranded DNAs).

### (1) Complex compound

In the method of the present invention, there can be used a complex compound, wherein a compound containing multiple phosphono groups (-P(O)(OH)₂) binds to a compound binding to a specific nucleic acid sequence via a linker portion containing an O-alkyloxime group (-O-N=).

With regard to the above-described complex compound, the number of phosphono groups in the compound containing multiple phosphono groups (-P(O)(OH)₂) is not limited, as long as it is plural. For example, the number of phosphono groups is preferably 2 to 10, and more preferably 3 to 6.

With regard to the above-described complex compound, the compound binding to a specific nucleic acid sequence is preferably an oligonucleotide or a peptide nucleic acid (PNA), for example. Such oligonucleotide includes DNA, RNA, and a derivative thereof. PNA is a polymer having an amide bond on the main chain and a nucleic acid base on the side chain. For example, PNA described on page 66 of "Seimei Kagaku no New Central Dogma (New Central Dogma of Life Science)" (Kagaku-dojin Publishing Company, INC; published in 2002) can be used. The number of bases of the oligonucleotide is preferably 7 to 50, and more preferably 10 to 20. On the other hand, the number of bases of the peptide nucleic acid is preferably 5 to 25, and more preferably 7 to 15.

In the above-described complex compound, the linker portion containing an O-alkyloxime group (-O-N=) connects a portion derived from the above-described compound containing multiple phosphono groups with a portion derived from the compound binding to a specific nucleic acid sequence. This O-alkyloxime group can be easily synthesized from O-alkylhydroxyamine and an aldehyde group in an aqueous solution (see the synthetic scheme of Figure 2). More specifically, a compound containing multiple phosphono groups, into which an aldehyde group has been introduced, and a compound binding to a specific nucleic acid sequence (an oligonucleotide, etc.), into which O-alkylhydroxyamine has been introduced, are prepared. Thereafter, the two compounds are allowed to react with each other, so that an O-alkyloxime group can be formed and at the same time, a complex compound can be generated via the aforementioned group. This complex compound can be easily prepared under conditions for the cleavage of a target nucleic acid. Moreover, the structure of an O-alkyloxime group has sufficiently high stability under conditions for the cleavage of a target nucleic acid in an aqueous solution. It results in high efficiency of cleaving a target nucleic acid, and it is also effective for reducing non-specific cleavage. It is to be noted that the structure of a linker portion containing such O-alkyloxime group is not limited to that shown in Figure 2. In the present invention, the concerned complex compound is a compound obtained for the first time using an O-alkyloxime group in a linker portion, which can be used for efficiently hybridizing a metal ion or the like serving as a nucleic acid-cleaving catalyst with a compound binding to a specific nucleic acid sequence.

A preferred example of the concerned complex compound is a compound represented by the following formula (1): 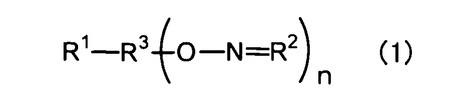 (wherein R¹ represents a compound binding to a specific nucleic acid sequence, R² represents a compound containing multiple phosphono groups, R³ represents a single bond or any given group, and n represents an integer of 1 to 10 (preferably, 3 to 6)).

Moreover, a complex compound, wherein, in the above-described formula (1), R¹ is an oligonucleotide or a peptide nucleic acid and R³ is a group represented by the following formula (3), is also preferable: 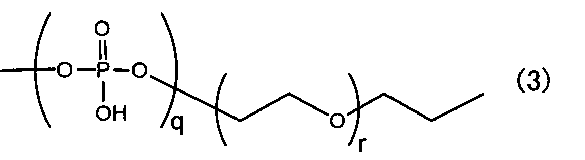 (wherein q represents an integer of 0 to 2 (preferably, 0 or 1), and r represents an integer of 0 to 30 (preferably, 0 to 10)).

Furthermore, a complex compound, wherein, in the above-described formula (1), R² is represented by the following formula (2), is also preferable: 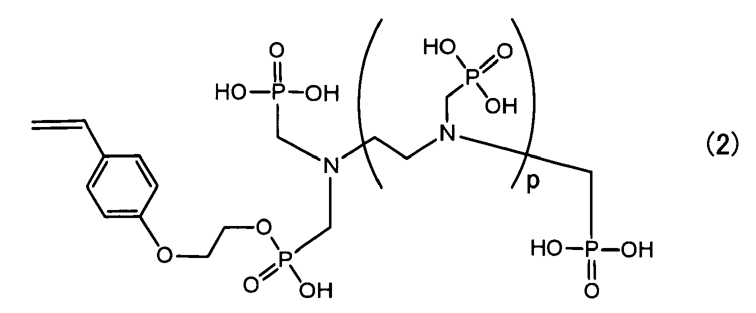 (wherein p represents an integer of 0 to 3 (preferably, 0 or 1)).

In the present invention, a complex compound, wherein, in the above-described formula (1), R¹ is an oligonucleotide or a peptide nucleic acid, R² is represented by the above-described formula (2), and R³ is a group represented by the above-described formula (3), is particularly preferable. Such complex compound includes the below-listed compounds, for example. (However, an oligonucleotide portion (SEQ ID NO: 1 or 2) in each of the listed complex compounds is provided for illustrative purpose only, and such oligonucleotide portion is not limited thereto.)

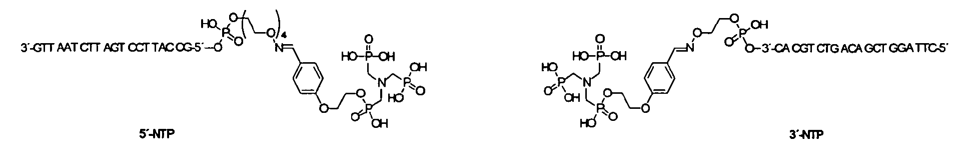

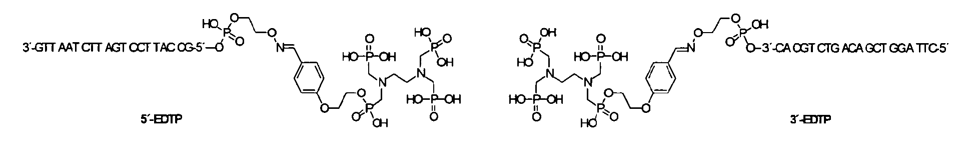

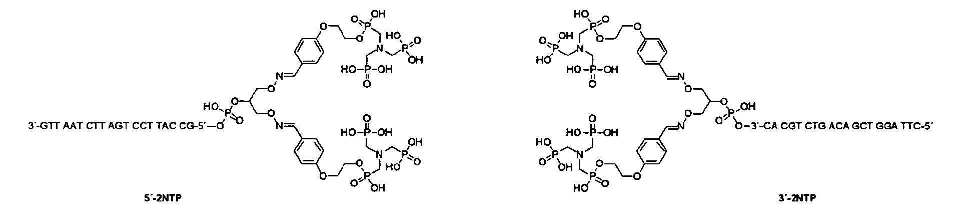

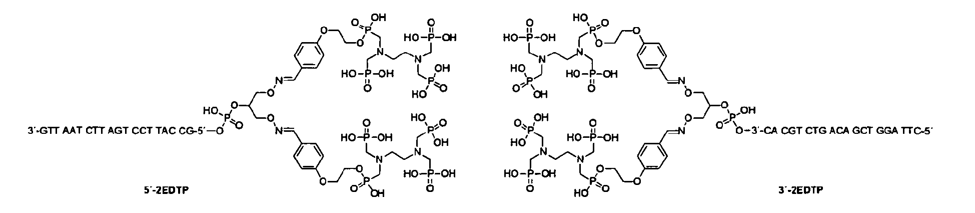

In a preferred embodiment of the complex compound of the present invention, a metal ion or a metal complex binds to multiple phosphono groups contained therein. Thus, using a complex compound that binds to a metal ion or the like, the non-specific cleavage of a nucleic acid can be greatly reduced, and cleavage efficiency and site-selectivity can be significantly improved. Moreover, when a target nucleic acid region is selectively cleaved in a cell, it is not necessary to independently introduce a complex compound and a metal ion or the like into the cell (in general, such introduction is extremely difficult), and the complex compound and the metal ion or the like may be introduced into the cell at one time. Accordingly, a nucleic acid in the cell can be easily cleaved. Furthermore, the amount of a metal ion or the like used as a nucleic acid-cleaving catalyst can be greatly reduced, and thus it is extremely preferable in terms of economic efficiency.

The types of the aforementioned metal ion and metal complex are not limited, as long as they have effects as catalysts for cleaving a nucleic acid. Preferred examples of such metal ion and metal complex include a cerium(IV) (Ce(IV)) ion, a cerium(IV) complex, a zirconium(IV) ion, a zirconium(IV) complex, a lanthanide(III) ion, and a lanthanide(III) complex. Of these, a cerium(IV) ion and a cerium(IV) complex are particularly preferable. Also, a cerium(III) ion and a cerium(III) complex are particularly preferable, as well as the cerium(IV) ion and the cerium(IV) complex. As stated above, in a reaction system for cleaving a target nucleic acid, such cerium(III) ion and cerium(III) complex react with oxygen or the like in the air, so that they are spontaneously oxidized to a cerium(IV) ion and a cerium(IV) complex, respectively, and as a result, they have effects as catalysts for cleaving a nucleic acid.

A cerium(III) ion and a cerium(III) complex hardly form a hydroxide and a precipitate thereof in a neutral solution and are homogenized, they are easily prepared as nucleic acid-cleaving molecules. In addition, since they do not become unnecessary gel to constitute a portion that is not directly associated with the cleavage of a nucleic acid, the prepared molecule is used nearly directly for the cleavage of a nucleic acid. Accordingly, the used amount can be kept extremely low, and thus it is economically advantageous. Further, unnecessary nucleic acid cleavage can be prevented in a reaction system. Also, such cerium(III) ion and a cerium(III) complex are greatly advantageous in that they are easily used in a cell.

When a cerium(IV) ion is allowed to bind to phosphono groups contained in the complex compound, the type of the cerium(IV) ion to be introduced into the reaction system is not limited, as long as it is an aqueous solution containing the cerium(IV) ion, such as a cerium(IV) diammonium sulfate aqueous solution, a cerium(IV) sulfate aqueous solution, an oxide of a cerium(III) chloride aqueous solution, an oxide of a cerium(III) sulfate aqueous solution, or an oxide of a cerium(III) perchlorate aqueous solution. Of these, cerium(IV) diammonium sulfate is preferable.

The type of a cerium(IV) complex is not limited. For example, a complex of cerium(IV) and polyamine-N-polycarboxylic acid is particularly preferable. Such complex of cerium(IV) and polyamine-N-polycarboxylic acid can be obtained by allowing cerium(IV) to come into contact with polyamine-N-polycarboxylic acid in water (other complexes are also obtained in the same manner). Herein, as polyamine-N-polycarboxylic acid, compounds described in *"*Kinzoku Chelate, I-IV (Metal Chelate, I-IV)" (Nankodo Co., Ltd.; published in 1967) can be used. Preferred examples include ethylenediaminetetraacetic acid, 1,3-diaminopropane-N,N,N',N'-tetraacetic acid, 1,4-diaminobutane-N,N,N',N'-tetraacetic acid, diethylenetriaminepentaacetic acid, and triethylenetetramine-N,N,N',N",N"',N"'-hexaacetic acid.

The above descriptions regarding the use of a cerium(IV) ion and a cerium(IV) complex may also be applied to the case of using a cerium(III) ion and a cerium(III) complex.

### (2) Method for cleaving target nucleic acid

(i) One embodiment of the method for cleaving a target nucleic acid of the present invention is a method for allowing a target nucleic acid to come into contact with a predetermined complex compound and a metal ion or a metal complex. Herein, the predetermined complex compound (except for a compound that binds to a metal ion or the like) is as described in the section 2(1) above.
(ii) Another embodiment of the method for cleaving a target nucleic acid of the present invention is a method for allowing a target nucleic acid to come into contact with a predetermined complex compound, to which a metal ion or a metal complex binds. Herein, the predetermined complex compound, to which a metal ion or a metal complex binds (a complex compound that binds to a metal ion or the like), is as described in the section 2(1) above.

In the cleavage methods described in (i) and (ii) above, when the metal ion or the metal complex is a cerium(III) ion or a cerium(III) complex, the cerium(III) ion or the cerium(III) complex is preferably oxidized before and/or after it is allowed to come into contact with the target nucleic acid, so as to convert it to a cerium(IV) ion or a cerium(IV) complex. Herein, the way of oxidizing the cerium(III) ion or the cerium(III) complex is not particularly limited. For example, spontaneous oxidation as a result of the reaction of cerium(III) with oxygen existing in the air is preferable.

Moreover, in the cleavage methods described in (i) and (ii) above, a complex compound (a) binding to a 5'-terminal region in the target portion of the target nucleic acid and a complex compound (b) binding to a 3'-terminal region in the target portion thereof can be used as the complex compounds. Herein, portions in the complex compound (a) and the complex compound (b), which bind to the target portions, are preferably both oligonucleotides or peptide nucleic acids. When such two types of complex compounds are used, it is preferable that a gap be present between the 5'-terminal region of the target nucleic acid, to which the complex compound (a) binds, and the 3'-terminal region of the target nucleic acid, to which the complex compound (b) binds, and that a desired cleavage point be present in the gap. Such structure makes it possible to cleave a nucleic acid at extremely high site-selectivity and efficiency. The present cleavage method is useful, when a target nucleic acid that is single-stranded DNA or one strand of double-stranded DNA is cleaved at a desired site.

Furthermore, in the cleavage methods described in (i) and (ii) above, the target nucleic acid is double-stranded, and a complex compound (A) binding to the target portion on one strand of the target nucleic acid and a complex compound (B) binding to the target portion on the other strand of the target nucleic acid can be used as the complex compounds. With regard to such two types of complex compounds, it is preferable that portions in the complex compound (A) and the complex compound (B), which bind to the target portions, have portions complementary to each other and also have portions that are not complementary to each other on the 5'-terminal and/or 3'-terminal sides thereof. Such structure makes it possible to effectively cleave a double-stranded target nucleic acid, and thus it is preferable. When such two types of complex compounds are used, portions in the complex compound (A) and the complex compound (b), which bind to the target portions, are oligonucleotides or peptide nucleic acids. Such portions are particularly preferably peptide nucleic acids. When the portions are peptide nucleic acids, each portion in the complex compound that binds to the target portion binds to each strand of the double-stranded target nucleic acid (hybridization), and at the same time, it invades the space between the two strands so as to loose the double strand (invasion), thereby partially forming a single-stranded region in each strand, as shown in Figure 3. A metal ion or a metal complex acts as a nucleic acid-cleaving catalyst on such single-stranded region, and as a result, the double-stranded target nucleic acid can be efficiently cleaved at a desired site.

### (3) Use of method for cleaving target nucleic acid

The method for cleaving a target nucleic acid of the present invention is a method capable of selectively cleaving an enormous nucleic acid strand at a desired site. Thus, the "barriers of nucleic acid size," which has conventionally been determined by the site-specificity of restriction enzyme, can be easily broken. That is to say, the product that can be accurately manipulated in genetic recombination using restriction enzyme is only plasmid DNA. Using the method of the present invention, however, not only a viral genome, but also the enormous nucleic acid strand of a higher organism can be selectively cleaved at a desired site, and accurate operations can be thereby performed. For instance, when the method of the present invention is used *in vitro,* enormous DNA or the like, which has not been easily manipulated by the conventional method, can be accurately manipulated. In addition, if the method of the present invention is used *in vivo* and it destroys an invading viral genome, it may provide an anti-viral agent. If the method of the present invention destroys a specific human gene (for example, a cancer gene), it may provide an effective cancer-treating agent. Further, an exchange reaction between similar genomes (homologous recombination) is promoted by cleaving a specific site in genomic DNA, so that the method of the present invention can be used to modify the genome.

Hence, useful applications of the method of the present invention are as follows: (a) the supply of a useful nucleic acid manipulation tool; (b) the development of a novel vector (genetic manipulation of adenovirus, various types of retroviruses, etc.); (c) an anti-viral agent with excellent targeting ability; (d) the development of an anticancer agent (destruction of telomere, etc.); and (e) promotion of homologous recombination (*in vivo* genetic recombination) (breeding, etc.). Herein, in (b) above, precise genetic manipulation is performed on adenovirus or the like, which has not ever been strictly genetically manipulated, so as to develop an excellent vector. In (c) to (e) above, the method of the present invention is utilized *in vivo,* for example. Moreover, in (c) above, the genome of virus that has invaded a living body is selectively destroyed. In (d) and (e) above, genomic DNA is cleaved at a desired site to achieve the object.

### 3. Reagent and kit for cleaving target nucleic acid

### (1) Target nucleic acid-cleaving reagent

(i) One embodiment of the target nucleic acid-cleaving reagent of the present invention is a target nucleic acid-cleaving reagent comprising a predetermined complex compound and a metal ion or a metal complex. Herein, the predetermined complex compound (except for a complex compound that binds to a metal ion or the like) is as described in the section 2(1) above.
(ii) Another embodiment of the target nucleic acid-cleaving reagent of the present invention is a target nucleic acid-cleaving reagent comprising a predetermined complex compound, to which a metal ion or a metal complex binds. Herein, the predetermined complex compound, to which a metal ion or a metal complex binds (a complex compound that binds to a metal ion or the like), is as described in the section 2(1) above.

In the reagents described in (i) and (ii) above, the above-described complex compound may include a complex compound (a) binding to a 5'-terminal region in the target portion of the target nucleic acid and a complex compound (b) binding to a 3'-terminal region in the target portion thereof. Herein, portions in the complex compound (a) and the complex compound (b), which bind to the target portions, are preferably both oligonucleotides or peptide nucleic acids. The present reagent is useful, when a target nucleic acid that is single-stranded DNA or one strand of double-stranded DNA is cleaved at a desired site.

In addition, in the reagents described in (i) and (ii) above, the target nucleic acid is double-stranded, and a complex compound (A) binding to the target portion on one strand of the target nucleic acid and a complex compound (B) binding to the target portion on the other strand of the target nucleic acid can be used as the complex compounds. In a case in which such two types of complex compounds are used, it is preferable that portions in the complex compound (A) and the complex compound (B), which bind to the target portions, have portions complementary to each other and also have portions that are not complementary to each other on the 5'-terminal and/or 3'-terminal sides thereof. Such structure makes it possible to effectively cleave a double-stranded target nucleic acid, and thus it is preferable. Herein, portions in the complex compound (A) and the complex compound (b), which bind to the target portions, are preferably both oligonucleotides or peptide nucleic acids. Such portions are particularly preferably peptide nucleic acids.

The reagent of the present invention may also comprise other ingredients as well as the above-mentioned substances. Such other ingredients are not limited.

### (2) Target nucleic acid-cleaving kit

The target nucleic acid-cleaving kit of the present invention comprises the above-described target nucleic acid-cleaving reagent of the present invention as a constituent. The kit of the present invention can be effectively used for the above-described method for cleaving the target nucleic acid of the present invention, and it is extremely highly useful and practical.

The kit of the present invention may comprise other constituents as well as the above-described constituent. Examples of such other constituents include various types of buffers, sterilized water, an Eppendorf tube, a nucleic acid coprecipitating agent, various types of gel (powders), an antiseptic such as sodium azide, and experiment manuals (instructions). The present kit may further comprise various types of electrophoresis apparatuses, as necessary.

Hereinafter, the present invention will be more specifically described in the following examples. However, these examples are not intended to limit the scope of the present invention.

### [Example 1]

### <Synthesis of complex compound of the present invention>

A phosphoroamidite monomer having a hydroxyl group protected with isophthalimide and a benzaldehyde derivative containing multiple phosphono groups (L1-NTP and L1-EDTP shown in the upper case of Figure 2, etc.) were both synthesized according to known synthesis methods described in publications.

As shown in the lower case of Figure 2, a phosphoroamidite monomer having a hydroxyl group protected with isophthalimide was allowed to react with a desired DNA oligomer synthesized using a DNA synthesizer, so as to obtain a compound (i). Thereafter, the compound (i) was treated with a hydrazine/pyridine/acetic acid mixture (1 : 32 : 8 (v/v/v)) to remove the isophthalimide group (reaction step a in the lower case of Figure 2), so as to obtain a compound (ii). The compound (ii) was allowed to react with a benzaldehyde derivative containing multiple phosphono groups (L1-NTP and L1-EDTP, etc.) in an aqueous solution, so as to form an O-alkyloxime group (-O-N=) between the thus reacted compounds (reaction step b or c in the lower case of Figure 2).

As a result, DNA derivatives (compounds (iii) and (iv)) containing multiple phosphono groups, which comprised a linker portion containing an O-alkyloxime group, were obtained as the complex compounds of the present invention.

### [Example 2]

### <Site-selective cleavage (1) of target DNA>

According to the method described in Example 1, various DNA derivatives containing multiple phosphono groups were synthesized as the complex compounds of the present invention. In the synthesized various DNA derivatives, DNA having the nucleotide sequence shown in the following SEQ ID NO: 1 or 2 was used as a DNA oligomer portion (the number of bases: 20) to be bound to target DNA.
5'-GCCATTCCTGATTCTAATTG-3' (SEQ ID NO: 1)
5'-CACGTCTGACAGCTGGATTC-3' (SEQ ID NO: 2)

The synthesized various DNA derivatives are shown below (5'-NTP (2), 3'-NTP (2), 5'-EDTP (3), 3'-EDTP (3), 5'-2NTP (4), 3'-2NTP (4), 5'-2EDTP (6), and 3'-2EDTP (6) (wherein the number described in each parentheses indicates the number of phosphono groups contained in a single molecule). DNA derivatives used in Examples 3 and 4 as described later are also shown below.

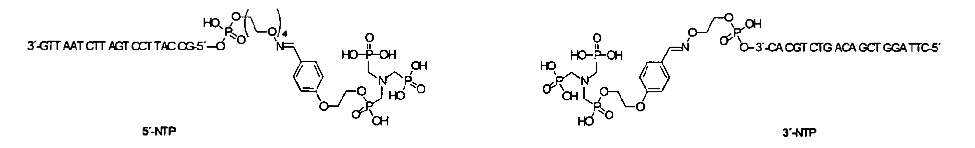

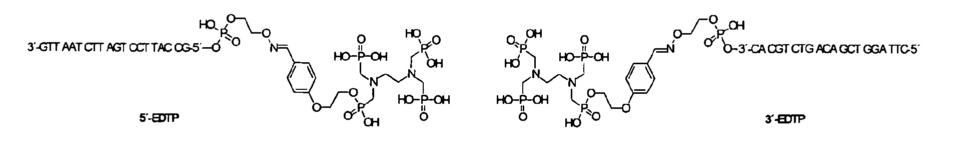

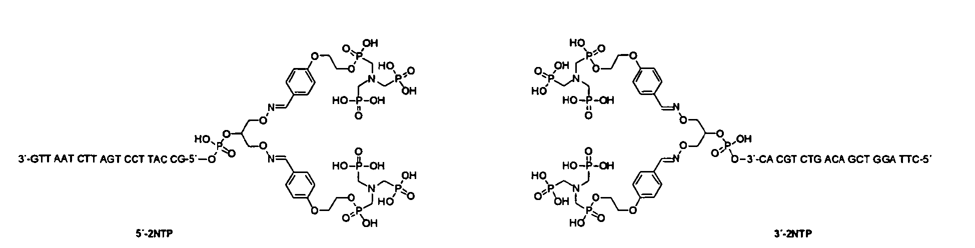

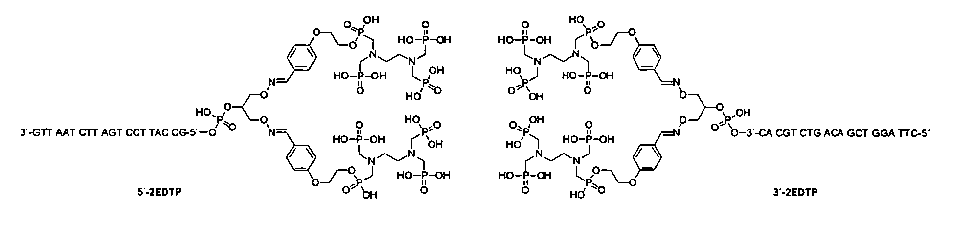

In addition, a DNA derivative having a phosphonate group (a single phosphonate group) at the terminus of a DNA oligomer thereof and an untreated DNA oligomer were also prepared as controls.

In a target DNA-cleaving reaction, two types of (possibly, one type of) the above-described DNA derivatives were first added to a 1 µM aqueous solution (5 mM HEPES buffer, pH 7, 100 mM NaCl) of target DNA with an FAM-labeled 5'-terminus (an oligonucleotide with the number of bases of 85: 5'-FAM-GAACTGGACCTCTAGCTCCTCAATTAGAATCAGGAATGGCTTATGGTGCAGA CTGTCGACCTAAGTAGACGCAATGTCGGACGTA-3' (SEQ ID NO: 3; the underlined portion indicates a region to which the DNA oligomer portion in each DNA derivative binds (hybridizes)), resulting in a final concentration of 2 µM in both cases of the two derivatives. Thereafter, the obtained mixture was heated at 90°C for 1 minute, and the temperature was then gradually decreased to room temperature, so as to form a double strand (hybridization), and a gap portion consisting of 5 bases was prepared in the target DNA. The embodiment of the experiment in which various DNA derivatives were used is as shown in a schematic view (B) on the right of Figure 4. Subsequently, after completion of the hybridization, a Ce(IV)/EDTA aqueous solution was added to the reaction system to a final concentration of 50 µM, and the obtained mixture was then reacted at 37°C for 114 hours. The cleavage of the target DNA was confirmed by 20% denatured polyacrylamide gel electrophoresis.

The results are as shown in a photograph (A) on the left of Figure 4. The details of each lane are as shown below.
Lane 1: Untreated
Lane 2: only a Ce(IV) · EDTA complex
Lane 3: Untreated double-stranded DNA
Lane 4: Double-stranded DNA, to the terminus of which a phosphonate group binds
Lane 5: Double-stranded DNA, to one strand of which an NTP group binds
Lane 6: Double-stranded DNA, to the other strand of which an NTP group binds
Lane 7: Double-stranded DNA, to both strands of which NTP groups bind
Lane 8: Double-stranded DNA, to one strand of which an EDTP group binds
Lane 9: Double-stranded DNA, to the other strand of which an EDTP group binds
Lane 10: Double-stranded DNA, to both strands of which EDTP groups bind

When NTP groups or EDTP groups were allowed to bind to both DNA oligomers of the two types of DNA derivatives, the target DNA was efficiently cleaved at a gap portion (lanes 7 and 10). In contrast, when an untreated DNA oligomer was used (lane 3), almost no cleavage of the target DNA was observed. When a phosphonate group was allowed to bind to the terminus of the DNA oligomer of the two types of DNA derivatives as well (lane 4), cleavage was observed only at an extremely weak level.

### [Example 3]

### < Site-selective cleavage (2) of target DNA>

Basically, the same operations as those described in Example 2 (Figure 4) were carried out with the exceptions that an oligonucleotide with the number of bases of 41 (5'-FAM-CAATTAGAATCAGGAATGGCNGTGCAGACTGTCGACCTAAG-3') (SEQ ID NO: 4; the underlined portion indicates a region to which the DNA oligomer in each DNA derivative binds (hybridizes)) was used, that the number of bases at a gap portion was 1, that the concentration of Ce(IV)/EDTA was 20 µM, and that the reaction time was 67 hours.

The results are as shown in a photograph (A) on the left of Figure 5 (the results of 20% denatured polyacrylamide gel electrophoresis).

When EDTP groups or 2EDTA groups were allowed to bind to both DNA oligomers of the two types of DNA derivatives (lanes 5, 8, 9, and 10), the target DNA was cleaved only almost at a single site (at a gap portion). When a 2EDTP group was allowed to bind to either one DNA oligomer of the two types of DNA derivatives (lanes 6 and 7), sufficiently effective cleavage was observed, although the obtained results were inferior to the results of lanes 5, 8, 9, and 10.

### [Example 4]

### < Site-selective cleavage (3) of target DNA>

According to the method described in Example 1, various DNA derivatives containing multiple phosphono groups were synthesized as the complex compounds of the present invention. Specifically, the DNA derivatives, 5'-EDTP (3) and 3'-EDTP (3) (wherein the number described in each parentheses indicates the number of phosphono groups contained in a single molecule), shown in Example 2, were synthesized. As in the case of Example 2, in the synthesized various DNA derivatives, DNA having the nucleotide sequence shown in the following SEQ ID NO: 1 or 2 was used as a DNA oligomer portion (the number of bases: 20) to be bound to target DNA.
5'-GCCATTCCTGATTCTAATTG-3' (SEQ ID NO: 1)
5'-CACGTCTGACAGCTGGATTC-3' (SEQ ID NO: 2)

In addition, as in the case of Example 2, a DNA derivative having a phosphonate group (a single phosphonate group) at the terminus of a DNA oligomer thereof and an untreated DNA oligomer were prepared as controls.

In a target DNA-cleaving reaction, two types of (possibly, one type of) the above-described DNA derivatives were first added to a 1 µM aqueous solution (5 mM HEPES buffer, pH 7, 100 mM NaCl) of target DNA with an FAM-labeled 5'-terminus (an oligonucleotide with the number of bases of 85: 5'-FAM-GAACTGGACCTCTAGCTCCTCAATTAGAATCAGGAATGGCTTATGGTGCAGA CTGTCGACCTAAGTAGACGCAATGTCGGACGTA-3' (SEQ ID NO: 3; the underlined portion indicates a region to which the DNA oligomer portion in each DNA derivative binds (hybridizes)), resulting in a final concentration of 1 µM in both cases of the two derivatives. Thereafter, the obtained mixture was heated at 90°C for 1 minute, and the temperature was then gradually decreased to room temperature, so as to form a double strand (hybridization), and a gap portion consisting of 5 bases was prepared in the target DNA. The embodiment of the experiment in which various DNA derivatives were used is as shown in a schematic view (B) on the right of Figure 6. Subsequently, after completion of the hybridization, a Ce(IV)/EDTA aqueous solution or a Ce(III) aqueous solution (specifically, a Ce(NO₃)₃ aqueous solution) was added to the reaction system to a final concentration of 4 µM, and the obtained mixture was then reacted at 50°C for 94 hours. During this reaction, the added Ce(III) was oxidized (spontaneously, with oxygen derived from the air) in the reaction system, and thereby, and it was thereby converted to Ce(IV). The cleavage of the target DNA was confirmed by 20% denatured polyacrylamide gel electrophoresis.

The results are as shown in a photograph (A) on the left of Figure 6. The details of each lane are as shown below.
Lane N: Untreated
Lane P4: only a Ce(IV) · EDTA complex
Lane P3: only Ce(III)
Lane 1: Double-stranded DNA, to both termini of which phosphonate groups bind
Lane 2: Single-stranded DNA, to which an EDTP group binds
Lane 3: Single-stranded DNA, to which an EDTP group binds
Lane 4: Double-stranded DNA, to both strands of which EDTP groups bind
Lane 5: Double-stranded DNA, to both strands of which phosphonate groups bind
Lane 6: Single-stranded DNA, to which an EDTP group binds
Lane 7: Single-stranded DNA, to which an EDTP group binds
Lane 8: Double-stranded DNA, to both strands of which EDTP groups bind (wherein a Ce(IV)/EDTA complex was added to lanes 1 to 4, and Ce(III) was added to lanes 5 to 8)

In the reaction systems to which Ce(IV)/EDTA was added (lanes 1 to 4), when EDTP groups were allowed to bind to both DNA oligomers of the two types of DNA derivatives (lane 4), the efficiency of cleaving the target DNA at a gap portion was significantly high.

In contrast, in the reaction systems to which Ce(III) was added (lanes 5 to 8), even when an EDTP group was allowed to bind to the DNA oligomer of one type of DNA derivative (lane 6), relatively high cleavage efficiency was observed. When EDTP groups were allowed to bind to both DNA oligomers of the two types of DNA derivatives (lane 8), cleavage efficiency much more higher than that in the case of adding Ce(IV)/EDTA (lane 4) was observed.

### INDUSTRIAL APPLICABILITY

According to the present invention, there can be provided a method for cleaving a target nucleic acid at a desired site using a metal ion or a metal ion complex as a catalyst for cleaving a nucleic acid (DNA, etc.), which has high site-selectivity, high reaction efficiency and low side-reactivity (non-specific reactivity), and is economical and convenient. Herein, in terms of economic efficiency, the used amount of a cleavage catalyst such as a metal ion can be significantly reduced, for example. In terms of convenience, this method is excellent in that a novel compound used for the above-mentioned method can be synthesized under conditions for a nucleic acid-cleaving reaction, for example. In these respects, the method for cleaving a target nucleic acid of the present invention is extremely useful and highly practical.

Moreover, according to the present invention, there can be provided a novel complex compound, a reagent, and a kit, which can be used for the above-described cleavage method of the present invention. As described above, the novel complex compound is significantly convenient in that it can be synthesized under conditions for a nucleic acid-cleaving reaction, and the structure of the synthesized complex compound is extremely stable under the same above conditions. Accordingly, the present complex compound is able to further improve its efficiency of cleaving a target nucleic acid. Sequence Listing Free Text
SEQ ID NO: 1 Synthetic DNA
SEQ ID NO: 2 Synthetic DNA
SEQ ID NO: 3 Synthetic DNA
SEQ ID NO: 4 Synthetic DNA
SEQ ID NO: 4 n indicates a, c, g, or t (position: 21)

## Claims

1. A complex compound, wherein a compound containing multiple phosphono groups binds to a compound binding to a specific nucleic acid sequence via a linker portion containing an O-alkyloxime group.

2. The complex compound according to claim 1, wherein the compound binding to a specific nucleic acid sequence is an oligonucleotide or a peptide nucleic acid.

3. The complex compound according to claim 2, wherein the number of bases of the oligonucleotide is 7 to 50 or the number of bases of the peptide nucleic acid is 5 to 25.

4. The complex compound according to any one of claims 1 to 3, wherein the nucleic acid is DNA.

5. The complex compound according to any one of claims 1 to 4, which is represented by the following formula (1): 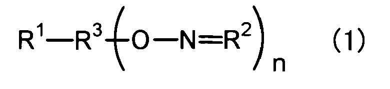 (wherein R¹ represents a compound binding to a specific nucleic acid sequence, R² represents a compound containing multiple phosphono groups, R³ represents a single bond or any given group, and n represents an integer of 1 to 10).

6. The complex compound according to claim 5, wherein R¹ is an oligonucleotide or a peptide nucleic acid, and R³ is a group represented by the following formula (3): 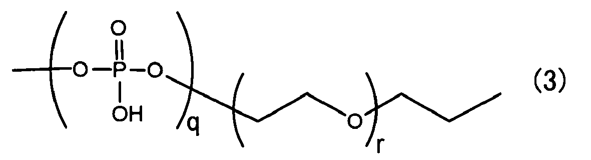 (wherein q represents an integer of 0 to 2, and r represents an integer of 0 to 30).

7. The complex compound according to claim 5 or 6, wherein R² is represented by the following formula (2): 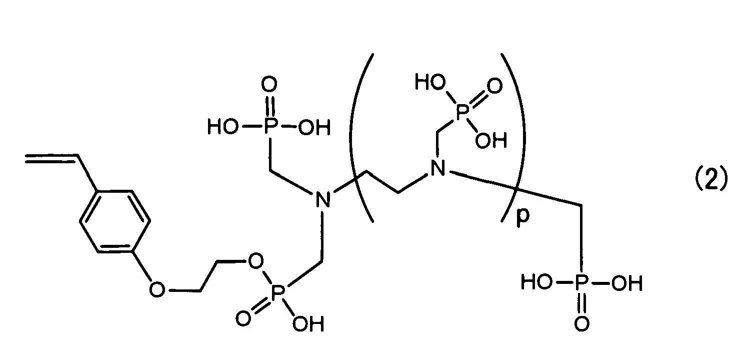 (wherein p represents an integer of 0 to 3).

8. The complex compound according to any one of claims 1 to 7, wherein a metal ion or a metal complex binds to the phosphono group.

9. The complex compound according to claim 8, wherein the metal ion or the metal complex is a cerium(IV) ion or a cerium(IV) complex, or a cerium(III) ion or a cerium(III) complex, respectively.

10. The complex compound according to claim 9, wherein the complex is a complex of cerium(IV) or cerium(III) and polyamine-N-polycarboxylic acid.

11. A method for cleaving a target nucleic acid, which comprises allowing a target nucleic acid to come into contact with the complex compound according to any one of claims 1 to 7 and a metal ion or a metal complex.

12. The method according to claim 11, wherein the metal ion or the metal complex is a cerium(IV) ion or a cerium(IV) complex, or a cerium(III) ion or a cerium(III) complex, respectively.

13. The method according to claim 12, wherein the complex is a complex of cerium(IV) or cerium(III) and polyamine-N-polycarboxylic acid.

14. The method according to claim 12 or 13, which comprises oxidizing the cerium(III) ion or the cerium(III) complex, before and/or after it is allowed to come into contact with the target nucleic acid, so as to convert it to a cerium(IV) ion or a cerium(IV) complex.

15. A method for cleaving a target nucleic acid, which comprises allowing a target nucleic acid to come into contact with the complex compound according to any one of claims 8 to 10.

16. The method according to claim 15, wherein the metal ion or the metal complex in the complex compound is a cerium(III) ion or a cerium(III) complex, and wherein the cerium(III) ion or the cerium(III) complex is oxidized before and/or after it is allowed to come into contact with the target nucleic acid, so as to convert it to a cerium(IV) ion or a cerium(IV) complex.

17. The method according to any one of claims 11 to 16, wherein the target nucleic acid is DNA.

18. The method according to any one of claims 11 to 17, wherein a complex compound (a) binding to a 5'-terminal region in the target portion of the target nucleic acid and a complex compound (b) binding to a 3'-terminal region in the target portion thereof are used as the complex compounds.

19. The method according to claim 18, wherein a gap is present between the 5'-terminal region of the target nucleic acid, to which the complex compound (a) binds, and the 3'-terminal region of the target nucleic acid, to which the complex compound (b) binds, and wherein a desired cleavage point is present in the gap.

20. The method according to claim 19, wherein portions in the complex compound (a) and the complex compound (b), which bind to the target portions, are oligonucleotides or peptide nucleic acids.

21. The method according to any one of claims 11 to 17, wherein the target nucleic acid is double-stranded, and a complex compound (A) binding to the target portion on one strand of the target nucleic acid and a complex compound (B) binding to the target portion on the other strand of the target nucleic acid are used as the complex compounds.

22. The method according to claim 21, wherein portions in the complex compound (A) and the complex compound (B), which bind to the target portions, are oligonucleotides or peptide nucleic acids.

23. The method according to claim 22, wherein portions in the complex compound (A) and the complex compound (B), which bind to the target portions, have portions complementary to each other and also have portions that are not complementary to each other on the 5'-terminal and/or 3'-terminal sides thereof.

24. A reagent for cleaving a target nucleic acid, which comprises the complex compound according to any one of claims 1 to 7 and a metal ion or a metal complex.

25. The reagent according to claim 22, wherein the metal ion or the metal complex is a cerium(IV) ion or a cerium(IV) complex, or a cerium(III) ion or a cerium(III) complex, respectively.

26. The reagent according to claim 25, wherein the complex is a complex of cerium(IV) or cerium(III) and polyamine-N-polycarboxylic acid.

27. A reagent for cleaving a target nucleic acid, which comprises the complex compound according to any one of claims 8 to 10.

28. The reagent according to any one of claims 24 to 27, wherein the complex compound includes a complex compound (a) binding to a 5'-terminal region in the target portion of the target nucleic acid and a complex compound (b) binding to a 3'-terminal region in the target portion thereof.

29. The reagent according to claim 28, wherein portions in the complex compound (a) and the complex compound (b), which bind to the target portions, are oligonucleotides or peptide nucleic acids.

30. The reagent according to any one of claims 24 to 27, wherein the target nucleic acid is double-stranded, and the complex compound includes a complex compound (A) binding to the target portion on one strand of the target nucleic acid and a complex compound (B) binding to the target portion on the other strand of the target nucleic acid.

31. The reagent according to claim 30, wherein portions in the complex compound (A) and the complex compound (B), which bind to the target portions, are oligonucleotides or peptide nucleic acids.

32. The reagent according to claim 31, wherein portions in the complex compound (A) and the complex compound (B), which bind to the target portions, have portions complementary to each other and also have portions that are not complementary to each other on the 5'-terminal and/or 3'-terminal sides thereof.

33. A kit for cleaving a target nucleic acid, which comprises the reagent according to any one of claims 24 to 32.
